# EUROPEAN PATENT SPECIFICATION

(11) **EP 2 832 317 B1**
(45) Date of publication and mention of the grant of the patent: **15.02.2017**
(21) Application number: 13178718.6
(22) Date of filing: 31.07.2013
(51) Int. Cl.: A61F 2/24, A61F 2/95

(54) **Implant delivery device for folding or unfolding a medical implant based on a knot**
Implantatausgabevorrichtung zum Einklappen oder Aufklappen eines medizinischen Implantats
Dispositif de pose d'implant permettant de plier ou de déplier un implant médical basé sur un noeud

(43) Date of publication of application: 04.02.2015
(73) Proprietor: Venus MedTech (HangZhou), Inc., Hangzhou, Zhejiang (CN)
(72) Inventor: Lim, Hou-Sen, 455234 Singapore (SG); Götz, Wolfgang, 93051 Regensburg (DE)
(74) Representative: Bobbert & Partner Patentanwälte PartmbB

(56) References cited:
- US-A1- 2006 265 055
- US-A1- 2012 109 280
- US-B1- 6 302 891

## Description

The present invention relates to a medical implant delivery device (also referred to as an apparatus or short hereinafter: delivery device) according to claim 1 for folding or unfolding an implant.

From WO 2011063972 A8, implants are known which may be folded or unfolded upon implantation by using one or several threads or filaments wound around the implant. Furthermore, corresponding apparatuses for folding and unfolding are known from WO 2011063972 A8, from US 2012/109280 A1, from US 6 302 891 B1 and from US 2006/265055 A1.

One object of the present invention is to suggest an apparatus or an implant delivery device for folding or unfolding a foldable and/or unfoldable implant by using a tension thread. Furthermore, an example of a suitable method for releasing the implant from the delivery device after implantation is provided.

This object may be solved by a device having the features of claim 1.

According to the present invention, a medical implant delivery device for folding or unfolding at least one medical implant by using at least one tension thread or a set of tension threads is suggested. The implant delivery device includes a foldable implant comprising or connected to at least one tension thread for folding the implant. It further includes a shaft having a reception or retaining area for receiving the implant. It also comprises a tensioning device for altering a shape of the foldable and/or unfoldable implant by at least one of the tension threads or by at least one first string connected to the tension thread(s).

At least one of the implant delivery device and the implant comprises a releasing string knotted to tension threads by at least one knot.

The knot is configured such that it can be released or opened upon pulling the releasing string.

In the following, the use of the expression "may be" or "may have", and so on, is to be understood synonymously with "in exemplary embodiments is" or "in exemplary embodiments has", respectively, and so on, and is intended to illustrate exemplary embodiments according to the present invention.

Whenever numerical values are mentioned herein such as "one", "two" and the like, they have to be understood as values representing the lower threshold of numerical ranges. A long as this does not result in a contradiction in the eyes of the skilled one, numerical values, such as "one" shall be understood as comprising also "at least one". This interpretation or understanding is as well encompassed by the present invention as the understanding according to which a numerical value such as "one" may be understood as "exactly one" whenever this appears technically possible to the skilled person. Both understandings are covered by the present invention. This applies to any numerical value stated herein.

The invention relates to a device as defined by the claims. Insofar as the terms "invention" and/or "embodiment" are used in the following, and/or features are presented as being optional, this should be interpreted in such a way that the only protection sought is that of the invention as claimed. In particular the described method for using the device is not part of the invention.

Exemplary embodiments according to the present invention are each also subject of dependent claims.

Exemplary embodiments according to the present invention may comprise one or more of the features named hereafter in any arbitrary combination.

In some exemplary embodiments according to the present invention, at least one of the implant delivery device and the implant further comprises a pulling string also knotted to tension threads by the knot.

In certain exemplary embodiments according to the present invention, the implant delivery device comprises a releasing mechanism allowing a user to pull, or selectively pull, the releasing string, in particularly in order to disengage, interrupt or cease the force transmission between the tension threads and the implant such that the implant cannot be folded any longer by means of the delivery device.

In some exemplary embodiments according to the present invention, the implant delivery device further comprises a mechanical reversing device arranged to reverse or amend the direction in which the releasing string extends.

In certain exemplary embodiments according to the present invention, the implant delivery device further comprises a balance limiter.

In some exemplary embodiments according to the present invention, the knot comprises at least, or exactly, two loops, wherein the first loop is inserted into the second loop and can be pulled out of the second loop upon or by pulling the pulling string.

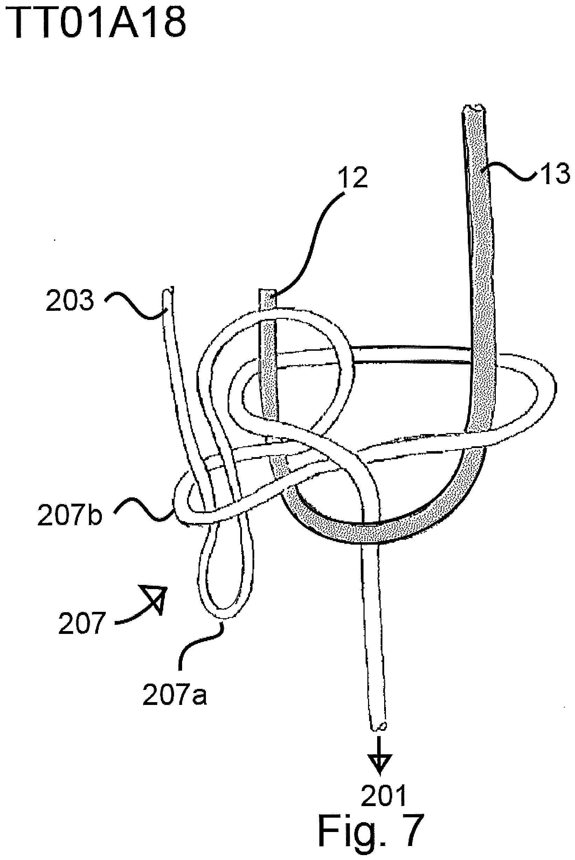

In certain exemplary embodiments according to the present invention, the knot is embodied or knotted as shown in Fig. 7.

In some exemplary embodiments according to the present invention, the implant is a stent or a cardiac valve assembly.

In certain exemplary embodiments according to the present invention, the set of tension threads comprises at least or exactly three tension threads each of which is preferably wound around at least parts of the implants periphery on at least two heights or longitudinal levels of the implant.

In some exemplary embodiments according to the present invention, the implant comprises a first guiding element and a second guiding element. The tension threads are each guided through or along at least sections of both the first and the second guiding elements.

In certain exemplary embodiments according to the present invention, at least one of the first guiding element and the second guiding element are hollow rings or channels that are preferably partly open in a cross section thereof.

In some exemplary embodiments according to the present invention, altering the shape of the implant means reducing or increasing a diameter, particularly an external diameter, of the implant. Such an alteration may or may not involve an alteration of the implant's length or any other kind of alteration.

In certain exemplary embodiments according to the present invention, folding the implant means reducing the diameter of the implant. Folding also covers "re-folding" of an once expanded implant.

In some exemplary embodiments according to the present invention, unfolding should be understood as increasing the diameter of the implant, or as expanding.

In certain exemplary embodiments according to the present invention, the diameter of the implant is arranged in the reception area in a plane perpendicular to a main flow direction of the implant in case fluids flow through the implant after its implantation.

In some exemplary embodiments of the apparatus according to the present invention, the at least one tension thread is a thread. The thread may be a surgical suture thread or similar to it. It may be a string or a wire. The thread may have the shape of a rope, a filament or a cord. The thread may be designed as a chain having a plurality of engaging chain links.

In the following, the term thread or tension thread may also define a plurality of threads or tension threads whenever a person skilled in the art recognizes the exchangeability of the terms.

In certain exemplary embodiments according to the present invention, the shaft of the apparatus is rigid. In some exemplary embodiments according to the present invention, the shaft of the apparatus is flexible in one or more directions (i.e., in a longitudinal direction or in a direction of the width of the shaft, respectively, in both directions or in other directions). In certain exemplary embodiments, the shaft is elongatable. In particular exemplary embodiments according to the present invention, the shaft is stiff.

In certain exemplary embodiments according to the present invention, the implant is permeable for fluids in its implanted state in its longitudinal direction. "Permeable" means that the fluid may flow through the implant, for example, through an inner lumen thereof.

In some exemplary embodiments of the apparatus according to the present invention, the implant is - at least temporarily - mounted or loosely arranged on or at the reception area of the apparatus at the moment of unfolding or folding. In some of these embodiments, the implant is arranged on or at the reception area or is interconnected with the reception area only by the tension threads.

In certain exemplary embodiments of the apparatus according to the present invention, the tensioning device includes at least one pulling string (or thread or wire or the like). The pulling string is arranged and/or provided in such a way that it may indirectly or directly apply a tension on the implant for altering the shape of the implant by the tension thread if the pulling string is pulled or activated by an operator (e. g., by the surgeon).

In certain exemplary embodiments of the apparatus according to the present invention, the pulling string is arranged and/or provided such that it may interact with the tension thread in order to transfer force or tension.

In some exemplary embodiment of the apparatus according to the present invention, the pulling string and the tension thread are intricate with each other at at least one site.

In certain exemplary embodiments according to the present invention, the term "intricate" is used to indicate that the tension thread is movable in at least one direction or in two directions relative to the pulling string.

According to some exemplary embodiments of the present invention, the term "movable" is to be understood as "slidable".

According to certain exemplary embodiments of the present invention, the term "intricate" means that the tension thread is movably arranged relative to the pulling string like a first link of a chain is movably arranged relative to an adjacent second link of this chain to which the first link is usually connected in a chain.

In some exemplary embodiments of the present invention, the term "intricate" shall indicate that the tension thread is simply crossed once with or wrapped around the pulling string or sections thereof.

In certain exemplary embodiments of the present invention, the transfer (or the transmittal, respectively) of force or tension between the pulling string and the tension thread is achieved by a non-form closure connection.

In some exemplary embodiments according to the present invention, the transfer of force or tension between the pulling string and the tension thread is achieved by a frictional connection.

In certain exemplary embodiments of the apparatus according to the present invention, the pulling string is embodied as at least one pulling thread or wire or consists of at least one pulling thread or wire.

In some exemplary embodiment of the apparatus according to the present invention, the tension thread and/or the pulling thread includes or constitutes at least one bundle or a plurality of threads or thread elements or consists thereof.

In some exemplary embodiments according to the present invention, the term "releasing mechanism or device" relates to a part of the apparatus for releasing the tension tread(s) from the implant, or the tension applied by the tension threads onto the implant, by unknotting the knot. The releasing device may be arranged to be operated by the surgeon.

In certain exemplary embodiments according to the present invention, an interior of the shaft is permeable or may be passed in at least sections thereof in the longitudinal direction of the shaft. The shaft has a wall, and the shaft includes at least one shaft aperture through which tension threads for folding and/or unfolding the implant may enter and/or exit the inner lumen of the shaft.

In certain embodiments according to the present invention a first string is connected to a multitude of tension threads, for example to - at least or exactly - three or six tension threads.

In some embodiments according to the invention, none of the tension threads is directly connected to the tensioning device of the implant delivery device, which may be a catheter, for altering a shape of the foldable and/or unfoldable implant. Rather, they are in direct contact with the first string. It is via the first string that they are in indirect contact with the tensioning device.

In certain exemplary embodiments according to the present invention, the method encompasses releasing the knot by pulling the pulling string. In some of these embodiments the tension thread may then be withdrawn from the implant, e. g. by the tensioning device or the surgeon himself.

In certain exemplary embodiments according to the present invention, the releasing device is a wire or a thread (or a multitude thereof, respectively).

In some exemplary embodiments according to the present invention, the releasing device is not embodied as a lock wire or lock thread that is withdrawn from the apparatus so as to allow the tension threads to be removed from the apparatus after final placement of the implant.

In certain exemplary embodiments according to the present invention, the releasing device does not comprise hooks and/or does not comprise rings for guiding or limiting tension strings or threads.

In some exemplary embodiments according to the present invention, the releasing device is embodied and/or intended to stay with the apparatus even after termination of the implantation of the implant.

In certain exemplary embodiments according to the present invention, the releasing device may be separated from the apparatus.

In some exemplary embodiments according to the present invention, the releasing device is embodied such that it is intended or configured to be used, according to the present invention, only within the patient's body.

In some exemplary embodiments of the apparatus according to the present invention, the releasing device is not a cutting device for cutting through the tension thread, and it does not comprise one.

In certain exemplary embodiments according to the present invention, the shaft of the apparatus is permeable or has a passage for fluids in its interior in at least some sections of its longitudinal direction. The shaft has a wall.

In some exemplary embodiments according to the present invention, the shaft includes at least one shaft aperture.

The at least one shaft aperture may preferably be arranged on a lateral area or on the circumference of the shaft rather than on the front side thereof.

In some exemplary embodiments of the present invention, the shaft of the apparatus includes a plurality of shaft apertures being uniformly or non-uniformly distributed along one or more circumferences and/or along the longitudinal extension of the shaft.

In certain exemplary embodiments according to the present invention tension threads for folding and/or unfolding the implant may enter and/or leave the apparatus through the shaft apertures.

In some exemplary embodiments according to the present invention, the releasing device exerts a tension on the knot via the releasing string substantially or exclusively in a longitudinal direction of the shaft.

In some exemplary embodiments according to the present invention, the releasing device enables a transition from the knotted state into the release or unknotted state.

In certain exemplary embodiments of the apparatus according to the present invention, said releasing device is arranged such that it enables a transition from the knotted state into the release or non-knotted sate independently from an operation of the tensioning device.

In certain exemplary embodiments according to the present invention, the set of tension threads comprises a self- balancing design. For more details regarding the idea of a self-balancing design it is referred to WO 2011063972 A8. See, in particular, Figs. 2 and 2A thereof.

In some exemplary embodiments according to the present invention, the apparatus is a catheter or a tip of a catheter that is provided to be interconnected with a catheter.

In certain exemplary embodiments according to the present invention, the method comprises altering the tension that is exerted on an implant by using at least one tension thread. The tension is preferably controlled by altering a length of the pulling string by which it extends out of the interior of the shaft or sections thereof.

In some exemplary embodiments according to the present invention, at least one of the apparatus, the implant and the set comprises exclusively, i.e. only, (one or more) materials that are MRI (short for: magnetic resonance imaging) compatible. In certain exemplary embodiments according to the present invention, at least one of the apparatus, the implant and the set comprises exclusively (one or more) materials that are not magnetic, ferromagnetic, or both. In some exemplary embodiments according to the present invention, at least one of the apparatus, the implant and the set does not comprise metal or any metal alloy.

In particular exemplary embodiments according to the present invention, the method comprises monitoring or controlling the position of the apparatus or the implant, or both, by means of magnetic resonance imaging (MRI) during or after implementation, advancing or delivering of the implant.

In certain exemplary embodiments according to the present invention, all instruments used for implanting or advancing the implant are MRI compatible.

Some or all advantages achievable by the apparatus according to the present invention may in certain exemplary embodiments of the present invention also be achieved by method according to the present invention.

What is said in here with regard to one tension thread holds also true for a multitude of tension threads whenever this does not stand in contrast to the general idea of the present invention.

In some embodiments according to the present invention, the shaft of the implant delivery device is arranged within the center of the implant at all times.

In certain embodiments according to the present invention, the implant is evenly folded or unfolded by the implant delivery device along its entire length (or longitudinal direction or extension).

Some or all exemplary embodiments according to the present invention may provide for one, several or all of the advantages named above and/or hereafter.

Among the advantages achievable according to the present invention is the opportunity of releasing threads from the implant by the releasing string that is arranged for ceasing the stated in which the thread(s) are knotted.

Further, the knot allows for a safe but temporary connection between the apparatus, and/or the implant, with the tension threads. At the same time, since the knot may be released after implantation, in some exemplary embodiments the present invention allows for an easy removal of the threads from the implant just by opening the knot and by pulling the thread out of the apparatus. In particular, for removing the threads from the implant or from the apparatus, no thread has to be unclamped or even cut. This contributes to a safe handling of the implant upon implantation since every device, such as a cutter, might fail, whereas in the absence of devices such a cutter with the present invention there are less devices that might fail. Also, providing a reliable mechanism such as a cutter always requires observing tolerances in the production process of the apparatus. In contrast, with the solution according to the present invention, less tolerances are to be observed since the knot is not a technical device which function relates on technical tolerances.

Providing at least one of the apparatus, the implant and the set to be MRI compatible allows advantageously for controlling the location and orientation of the apparatus or the implant, or both, by MRI upon use of the apparatus or implantation of the implant. No heat, sparks or artefacts are generated during MRI because of the materials chosen for the apparatus or the implant.

Another advantage achievable according to the present invention is the opportunity of independently actuating tension threads. This is due to the self balancing design provided in which the pulling string is entangled with the first string or wound around without being fixed to it. This way, the first string may move forth and back within the loop of the pulling string. Thus, it is possible to specifically fold and/or unfold implants having a first and a second tension thread, or a plurality of tension threads, which act on different parts or sections of the implant upon folding the latter. Parts or sections of the implant can thus be folded or unfolded though other parts or sections of the implant have already been completely folded or unfolded. This can i. a. be reasonable when an unfolded implant and the implantation site do not completely match in their dimensions, or if the implant does, e. g., not have a uniform shape or implantation space over its entire length.

Further, the knot allows for a very easy release of the tension threads.

In contrast to cutting, for example, knotting does not require the use of tension threads that provide sufficient resistance to the cutter blade. Rather, for knotting any tension thread will do, in particular also the very flexible ones which provide additional advantages by themselves. Among those advantages, a flexible tension thread may be withdrawn from the implant once it has been release without unintentionally crimping or folding the implant again (because of the resistance between the tension thread and the implant) that can be observed when a stiff(er) tension thread is withdrawn from the implant.

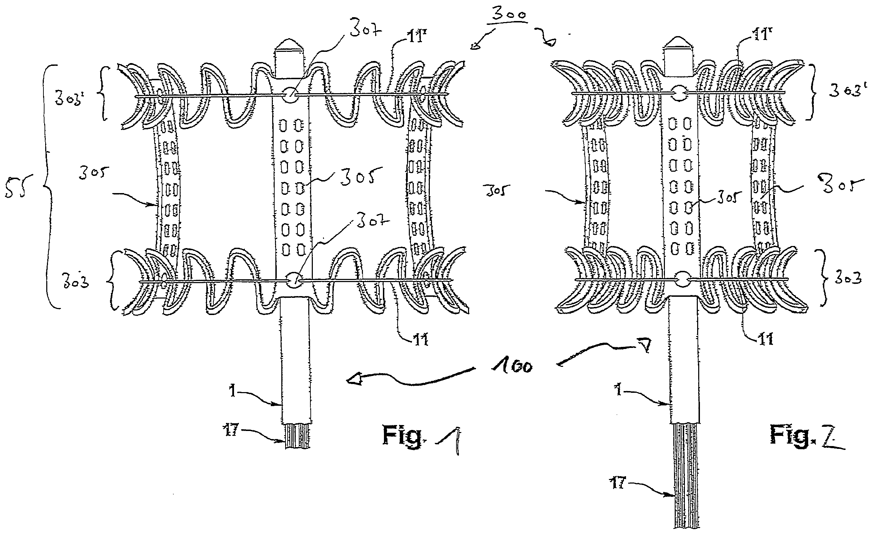

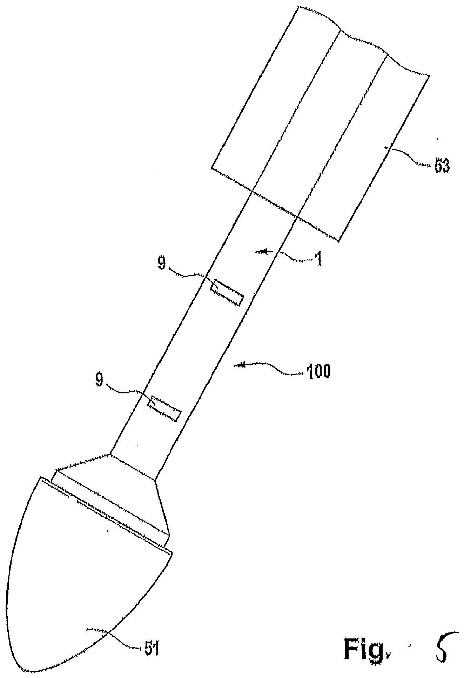

The knot suggested in Fig. 7 is designed in a way such that the tension thread's ends are bent at a minimum. This has the advantage that the ends of the tension threads are crinkled as little as possible so that they can be easily withdrawn through the apertures of both the implant and the delivery device, see, e. g. Figs. 1 and 5.

Also, the solution according to the present invention which is based on a knot allows using implants comprising tensions threads connected by a knot with all kinds of delivery devices. In particular, the features of the tension threads that come along with the implant do not have to match with a cutter or a clamping device already arranged on the delivery device since the cutter or the clamping device will simply not be used.

If, as contemplated in certain embodiments according to the present invention, one particular tension thread is wound around several sections of the implant, whereas the sections are at different longitudinal heights of the implant, and if both ends of that tension thread are tightened in the knot, then the crimping of the implant may be carried out in a very smooth and easy manner since the tension threads are pulled from both ends. At the same time, the crimping force is applied to the implant in a very homogeneous manner resulting in a very homogeneous folding.

Also, using a knot instead of a clamping device, a cutter or the like advantageously allows to build a delivery device which is smaller (e. g., in diameter) than prior art devices comprises such a releasing device (i. e. cutter, clamp, and the like).

Furthermore, using a knot having two loops as disclosed supra and infra means very little slippage which makes the knot a particularly safe connection.

In the following, examples of the present invention will be described with reference to the accompanying figures wherein similar or identical assemblies or elements are denoted by same reference numbers.
- **Fig. 1**: shows schematically simplified and in part section an apparatus according to the present invention with an expanded implant according to a first exemplary embodiment of the present invention;
- **Fig. 2**: shows the apparatus of Fig. 1 with the implant in a further (partly) folded condition;
- **Fig. 3**: shows the tip of an apparatus according to the present invention shown in a closed condition prior to implantation;
- **Fig. 4**: shows the tip of the apparatus according to the present invention as in Fig. 3 prior to implantation with partially withdrawn outer sleeve;
- **Fig. 5**: shows the tip of an apparatus according to the present invention as in Fig. 4 without implant;
- **Fig. 6**: shows a set of the delivery device comprising a knot; and
- **Fig. 7**: shows the knot of Fig. 6 in more detail.

**Fig. 1** shows schematically simplified and in part section an medical implant delivery device/an apparatus 100 according to the present invention with an expanded implant 300 according to a first exemplary embodiment of the present invention (the combination of apparatus 100 and implant 300 also being referred to as "set" herein).

A first tension thread 11 and a second tension thread 11' are arranged around the implant 300. As can be seen from Fig. 1, the implant 300 comprises a first guiding structure 303 for guiding the first tension thread 11 and a second guiding structure 303' for guiding the second tension thread 11'.

In the exemplary embodiment of Fig. 1, the first guiding structure 303 and the second guiding structure 303' are designed as rings or channel-like ring structures. These structures are optionally radially open but medially closed as it is exemplarily also shown in Fig. 1.

Also by way of example, two, three or more posts 305 are arranged between the first guiding structure 303 and the second guiding structure 303'. The posts 305 each comprises one, two or more openings 307 for letting pass the first or second tension threads 11, 11' from an inside of the implant 300 to on outside thereof.

The posts 305 may be configured to keep the distance between the first guiding structure 303 and the second guiding structure 303'.

In the example of Fig. 1, the threads 11 and 11' are provided for holding the implant 300 with regards to the apparatus 100. In any case, the diameter of the implant 300 or its folding state may be altered by varying the tension of the threads 11 and 11' as will be explained in more detail below.

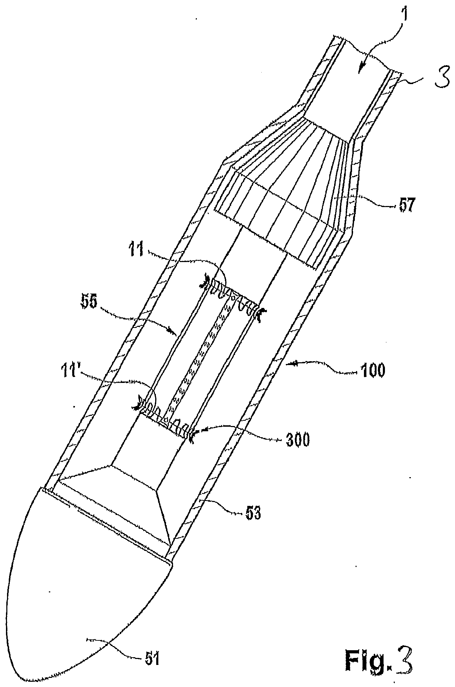

The apparatus 100 has a shaft 1 having a lumen covered by a wall (depicted with reference numeral "3" in, e.g., Figs. 3 and 13). In the lower area of Fig. 1, the wall of shaft 1 is longitudinally cut. Pulling threads 17 arranged within the lumen of the shaft 1 extend therefrom.

The pulling threads 17 are integral with or interconnected to threads 11 and 11' which are guided along the circumference of implant 300 at different levels - by the first and the second guiding structure 303 and 303' - thereof such that pulling or releasing the pulling threads 17 makes the threads 11 and 11' to exert more or less force on the implant 300 as it is also described in the patent application published under WO 2011/063972 Al. This way, operating the pulling threads 17 may provide for a change in one or more cross- section dimensions of the implant 300.

The threads 11 and 11' enter into the lumen of shaft 1 by apertures 9 not shown in Fig. 1 (but shown in Fig. 5) and they exit shaft 1 from such apertures again.

The expansion of implant 300 may benefit in the present exemplary embodiment from the internal stress or from shape- memory capacities of implant 300. The implant 300 may be manufactured from Nitinol or comprise such material. In order to expand the implant 300, the pulling threads 17 need, however, to be sufficiently released. For folding the implant 300 again, the pulling threads 17 are tightened again.

In Fig. 1, the longitudinal direction of the implant 300 extends from the bottom of Fig. 1 to its top or vice versa.

**Fig. 2** shows the apparatus 100 of Fig. 1. The implant 300 is in a partly folded condition (also referred to herein as "folded" or "refolded"). Since folding of the implant 300 has to be achieved by pulling the pulling threads 17, in Fig. 2 the pulling threads 17 protrude further out of shaft 1 than in Fig. 1.

In Fig. 1 (and likewise in Fig. 2), apparatus 100 is shown with only one upper ("second") thread 11' and one lower ("first") thread 11. This reduction (simplification) is used for improved clarity. It is therefore clear that any arbitrary number of upper and lower threads 11 and 11' may be provided ("upper" and "lower" relate to the upright position of the implant shown in Fig. 2). A corresponding number of apertures 9 may be provided.

**Fig. 3** shows an apparatus 100 according to the present invention with an implant 300 attached at or within an apparatus 100 according to the present invention.

Fig. 3 shows in part section a tip 51 of an apparatus 100 according to the present invention in a closed condition prior to implantation.

Shown in part section is an outer protective sleeve 53 which gives protection to a retaining area 55 for the implant 300. In the example of Fig. 3, the implant 300 is a stent which is arranged between the tip 51 and a collar 57. The collar 57 may advantageously guide the sleeve over the implant 300 which may be, e. g., a crimped stent, as in the example of Fig. 3.

The implant 300 is held by the threads 11 and 11' in a restrained or folded state in which it is not expanded.

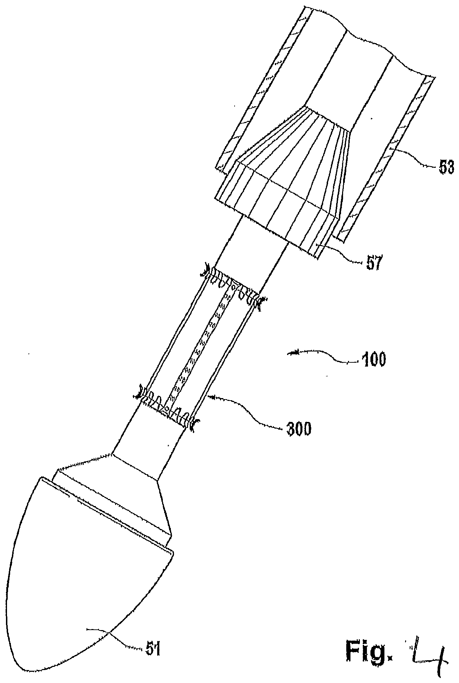

**Fig. 4** shows the tip 51 of Fig. 3 prior to implantation. The outer protective sleeve 53 is partly withdrawn. By withdrawing the outer protective sleeve 53, which is only provided by way of example, the implant 300 is ready for implantation. The restrained state is still maintained, substantially or fully by the tension of the circumferentially wound threads 11 and 11'.

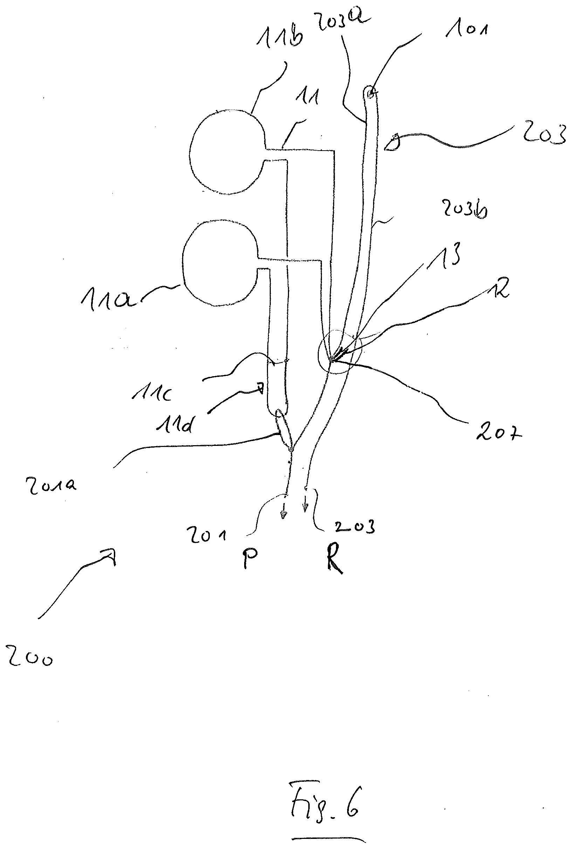

**Fig. 5** shows the tip 51 of the apparatus 100 of Fig. 4 without the implant 300. In Fig. 6, the wall apertures or shaft apertures 9 through which the threads 11 and 11' - which are also not shown in Fig. 5 - exit and enter shaft 1.

Shaft 1 features an arbitrary number of apertures 9, at one, two (as shown in Fig. 5) or more longitudinal heights of the axis.

The shaft apertures 9 extend through the entire thickness of the wall of shaft 1 and, hence, interconnect the lumen or inner space of shaft 1 with the exterior of shaft 1.

The shaft apertures 9 may be evenly spaced from each other around the circumference of shaft 1. Alternatively, they may be divided with at least two different distances from each other around the circumference.

**Fig. 6** shows a set 200 of the delivery device 100 (the device 100 is not shown in Fig. 6). The set 200 comprises a first tension thread 11. The first tension thread 11 comprises two loops 11a and 11b indicating that the one tension thread 11 is wound in two loops 11a, 11b around the implant (not shown in Fig. 6), preferably through the first and second guiding structures 303, 303'.

The tension thread 11 comprises a first end section 12 and a second end section 13.

The set 200 further comprises a pulling string or pulling thread 201. Pulling it results in a folding of the implant 300 by reducing the loops' 11a, 11b diameters.

The pulling string 201 is interconnected with the tension thread 11. This may be by an optionally provided interconnecting loop 201a, the knot 207, another knot, or the like. Also, the pulling string 201 and the tension thread 11 may be intricated with each other.

The set 200 further comprises a releasing string 203. In the exemplary embodiment of Fig. 6, the releasing string 203 comprises an upwardly directed section 203a and a downwardly directed section 203b. Between them there is a reversing device 101, or redirector, which is an element of the delivery device 100. The reversing device 101 is part of the apparatus 100 and arranged to redirect the effect of the releasing string 203 when pulling it.

As can be seen in Fig. 6, the set 200 further comprises a knot 207. By the knot 207, both ends 12, 13 of the tension thread 11 are interconnected to the pulling string 201 such that pulling the pulling string 201 may result also in pulling the tension thread 11. For details regarding the knot 207, it is referred to Fig. 7. As can be seen there, the releasing string 203 is also part of the knot 207.

The releasing string 203 is configured such that it unties the knot 207 if it is sufficiently strong pulled in the direction indicated by the arrow R in Fig. 6. Pulling may be effected by hand or by any suitable device that is part of the implant delivery device.

The pulling string 17 is tied to at least one of the first and the second end sections 12, 13 of the tension thread 11 by the knot 207.

In the exemplary embodiment shown in Fig. 6, both the first and the second end sections 12, 13 of the tension thread are entangled in a knot 207. In other embodiments, only one end 12, 13 is entangled by the knot 207.

Also, in Fig. 6, only one tension thread 11 is shown. However, instead of only one tension thread 11 the set 200 may comprise a multitude of tension threads. In that case, they all might be tangled with one knot 207. Alternatively, the multitude of tension threads might as well be tied by more that one knot 207. In case of several knots, they might all be identical or similar to the one shown in Figs. 6 or 7. However, several knots might, of course, be knotted in different ways.

In the exemplary embodiment of Fig. 6, the tension thread 11 comprises a balance limiter 11c. The balance limiter 11c may be fixedly connected at two sites with the tension thread 11 defining a loop 11d of the tension thread 11 of fixed length. That way, after the knot 207 is released, the balance limiter 11c is there to allow the tension thread 11 to be retrieved completely from the catheter by pulling on 201. It may ensure that the tension thread 11 is pulled out from the implant in a desired manner. The balance limiter 11c may be a simple string interconnecting the two halfs or arms or the like of the tension thread 11. It may be a knot between the two halfs or arms.

The pulling string 201 is connected fixedly to the tension thread 11 such that after untying the knot 207 the then loosened tension thread 11 may be separated from the implant 300 by pulling the pulling string 201. The pulling string 201 may be connected with the tension thread 11 not only in the knot 207 but additionally, directly or as shown in Fig. 6 in an indirect manner, e. g. by means of the loop 201a or any other section of the pulling string 201. Since the pulling string 201 is connected to the tension thread 11, the latter may be withdrawn from the implant 300 when the pulling string 201 is withdrawn by the user.

In one exemplary embodiment, as much as three tension threads are provided in the identical way as the tension thread 11.

Similarly, more than one pulling string 201, more than one releasing string 203 and more than one knot 207 may be provided.

Also, the two loops 11a, 11b of tension thread 11 may well be distributed to two separate tension threads, one comprising loop 11a, the other comprising loop 11b.

Finally, in certain embodiments according to the present invention, as much as, e. g., three or six knots 207 are provided.

**Fig. 7** shows an exemplary knot 207 in more detail.

As can be seen in Fig. 7, the knot 207 comprises a first loop 207a which enters and returns from a second loop 207b. The first loop 207a extends into or is part of the releasing string 207.

The knot 207 is configured to tighten if one pulls the pulling string 201 and to untie if one pulls the releasing string 203.

### Reference numerals

- 100: apparatus or medical implant delivery device
- 101: reversing device

- 200: set
- 201: pulling string
- 201a: loop
- 203: releasing string
- 203a: releasing string - downward section
- 203b: releasing string - upward section
- 207: knot

- 300: implant
- 303: first guiding structure of the implant
- 303': second guiding structure of the implant
- 305: post
- 307: opening

- 1: shaft
- 3: wall of shaft 1
- 9: apertures
- 11: tension thread(s)
- 11a: first loop
- 11b: second loop
- 11c: balance limiter
- 11d: loop
- 11': second tension thread(s)
- 12: first end section of tension thread 11
- 13: second end section of the tension thread 11
- 14: interconnecting loop
- 17: pulling string or thread
- 51: tip
- 53: external protective sleeve
- 55: reception/retaining area
- 57: collar

## Claims

1. A medical implant delivery device (100) for folding or unfolding at least one medical implant (300) by using at least one tension thread (11, 11') or a set (20₀) of tension threads, wherein the implant delivery device (100) includes:
- a foldable implant (300) comprising at least one tension thread (11, 11') for folding the implant (300);
- a shaft (1) including a reception or retaining area (55) for receiving the implant (300);
- a tensioning device for altering a shape of the foldable and/ or unfoldable implant (300) by at least one of the tension threads (11, 11') or by at least one first string connected to the tension threads (11, 11');
**characterized in that** at least one of the implant delivery device (100) and the implant (300) comprises a releasing string (203) knotted to tension threads (11, 11') by at least one knot (207);
wherein the knot (207) is configured such that it can be released or opened upon pulling the releasing string (203), wherein the knot (207) comprises at least or exactly two loops (207a, 207b),
wherein the first loop (207a) is inserted into the second loop (207b); wherein the knot (207) is embodied or knotted as shown in Fig. 7; and
wherein at least one of the implant delivery device (100) and the implant (300) further comprises a pulling string (201) also knotted to tension threads (11, 11') by the knot (207).

2. The implant delivery device (100) according to claim 1, comprising a releasing mechanism allowing a user to pull or selectively pull the releasing string (203).

3. The implant delivery device (100) according to claim 1 or 2, further comprising a mechanical reversing device (101) arranged to reverse or amend the direction in which the releasing string (203) extends.

4. The implant delivery device (100) according to anyone of the preceding claims, further comprising a balance limiter (11c).

5. The implant delivery device (100) according to anyone of the preceding claims, wherein the implant (300) is a stent or a cardiac valve assembly.

6. The implant delivery device (100) according to anyone of the preceding claims, wherein the set (200) of tension threads comprises at least or exactly three tension threads (11, 11') each of which is wound around at least parts of the implants periphery on at least two longitudinal levels of the implant (300).

7. The implant delivery device (100) according to anyone of the preceding claims, wherein the implant (300) comprises a first guiding element (303) and a second guiding element (303'), wherein the tension threads (11, 11') are each guided through at least sections of both the first and the second guiding elements (303, 303').

8. The implant delivery device (100) according to claim 7, wherein at least one of the first guiding element (303) and the second guiding element (303') are rings or channels that are partly open in a cross section thereof.

## Patentansprüche

1. Medizinimplantateinsetzvorrichtung (100) zum Falten oder Entfalten von mindestens einem medizinischen Implantat (300) durch Verwendung von mindestens einem Spannfaden (11, 11') oder einem Satz (200) von Spannfäden, wobei die Implantateinsetzvorrichtung (100) umfasst:
- ein faltbares Implantat (300) umfassend mindestens einen Spannfaden (11, 11') zum Falten des Implantats (300);
- einen Schaft (1) einschließlich eines Aufnahme- oder Haltebereichs (55) zum Aufnehmen des Implantats (300);
- eine Spannvorrichtung zum Ändern der Form des faltbaren und/oder entfaltbaren Implantats (300) durch mindestens einen der Spannfäden (11, 11') oder durch mindestens einen ersten Strang, der mit den Spannfäden (11, 11') verbunden ist;
**dadurch gekennzeichnet, dass** mindestens eine(s) aus der Implantateinsetzvorrichtung (100) und dem Implantat (300) einen Freisetzstrang (203) umfasst, der durch mindestens einen Knoten (207) mit den Spannfäden (11, 11') verknotet ist;
wobei der Knoten (207) derart konfiguriert ist, dass er freigesetzt oder geöffnet werden kann durch Ziehen an dem Freisetzstrang (203), wobei der Knoten (207) mindestens oder genau zwei Schlaufen (207a, 207b) umfasst, wobei die erste Schlaufe (207a) in die zweite Schlaufe (207b) eingesetzt ist;
wobei der Knoten (207) ausgeführt oder geknotet ist wie in Fig. 7 gezeigt ist; und wobei mindestens eine(s) aus der Implantateinsetzvorrichtung (100) und dem Implantat (300) zudem einen Ziehstrang (201) umfasst, der ebenfalls mit den Spannfäden (11, 11') durch den Knoten (207) verknotet ist.

2. Die Implantateinsetzvorrichtung (100) nach Anspruch 1, einen Freisetzmechanismus umfassend, der es einem Benutzer gestattet, den Freisetzstrang (203) zu ziehen oder gezielt zu ziehen.

3. Die Implantateinsetzvorrichtung (100) nach Anspruch 1 oder 2, zudem eine mechanische Umkehrvorrichtung (101) umfassend, die angeordnet ist, um die Richtung zu ändern oder umzukehren, in der der Freisetzstrang (203) ausfährt.

4. Die Implantateinsetzvorrichtung (100) nach einem der vorhergehenden Ansprüche, die zusätzlich einen Balancebegrenzer (11 c) umfasst.

5. Die Implantateinsetzvorrichtung (100) nach einem der vorhergehenden Ansprüche, wobei das Implantat (300) ein Stent oder eine Herzklappenanordnung ist.

6. Die Implantateinsetzvorrichtung (100) nach einem der vorhergehenden Ansprüche, wobei der Satz (200) von Spannfäden mindestens oder genau drei Spannfäden (11, 11') umfasst, von denen jeder zumindest um Teile der Implantatperipherie auf mindestens zwei Längsebenen des Implantats (300) gewunden ist.

7. Die Implantateinsetzvorrichtung (100) nach einem der vorhergehenden Ansprüche, wobei das Implantat (300) ein erstes Führungselement (303) und ein zweites Führungselement (303') umfasst, wobei die Spannfäden (11, 11') jeweils durch mindestens Teile sowohl der ersten und der zweiten Führungselemente (303, 303') geführt werden.

8. Die Implantateinsetzvorrichtung (100) nach Anspruch 7, wobei mindestens eines des ersten Führungselements (303) und des zweiten Führungselements (303') Ringe oder Kanäle sind, die in einem Querschnitt teilweise offen sind.

## Revendications

1. Un dispositif de délivrance d'implant médical (100) pour plier ou déplier au moins un implant médical (300) en utilisant au moins un fil de tension (11, 11') ou un ensemble (200) de fils de tension, le dispositif de délivrance d'implant (100) comprenant :
- un implant pliable (300) comprenant au moins un fil de tension (11, 11') destiné à plier l'implant (300);
- une tige (1) comprenant une zone (55) de réception ou de retenue destinée à recevoir l'implant (300);
- un dispositif de tension destiné à modifier la forme de l'implant (300) pliable et / ou dépliable au moyen de l'un au moins des fils de tension (11, 11') ou au moyen d'au moins une première chaîne connectée aux fils de tension (11, 11');
**caractérisé en ce qu'**au moins l'un du dispositif de délivrance d'implant (100) et de l'implant (300) comprend une chaîne de libération (203) nouée aux fils de tension (11, 11') au moyen d'un noeud (207);
où le noeud (207) est configuré de telle sorte qu'il peut être libéré ou ouvert en tirant sur la chaîne de libération (203) et comprend au moins ou exactement deux boucles (207a, 207b), la première boucle (207a) étant insérée dans la seconde boucle (207b) ;
le noeud (207) étant formé ou noué comme représenté sur la figure 7 ; et
et où l'un au moins du dispositif de délivrance d'implant (100) et de l'implant (300) comprend de plus une chaîne d'entrainement (201) nouée elle aussi aux fils de tension (11, 11') au moyen du noeud (207).

2. Le dispositif de délivrance d'implant (100) selon la revendication 1, comprenant un mécanisme de libération permettant à un utilisateur de tirer ou de tirer de manière sélective la chaîne de libération (203).

3. Le dispositif de délivrance d'implant (100) selon la revendication 1 ou 2, comprenant en outre un dispositif d'inversion mécanique (101) agencé de façon à inverser ou à modifier la direction dans laquelle la chaîne de libération (203) s'étend.

4. Le dispositif de délivrance d'implant (100) selon l'une quelconque des revendications précédentes, comprenant en outre un limiteur d'équilibrage (11c).

5. Le dispositif de délivrance d'implant (100) selon l'une quelconque des revendications précédentes, dans lequel l'implant (300) est un stent ou un arrangement de valve cardiaque.

6. Le dispositif de délivrance d'implant (100) selon l'une quelconque des revendications précédentes, dans lequel l'ensemble (200) de fils de tension comprend au moins ou exactement trois fils de tension (11, 11') étant, chacun, enroulé autour d'au moins une partie de la périphérie de l'implant sur au moins deux niveaux longitudinaux de l'implant (300).

7. Le dispositif de délivrance d'implant (100) selon l'une quelconque des revendications précédentes, dans lequel l'implant (300) comprend un premier élément de guidage (303) et un second élément de guidage (303'), les fils de tension (11, 11') étant guidés, chacun, au travers de sections du premier et du second élément de guidage (303, 303').

8. Le dispositif de délivrance d'implant (100) selon la revendication 7, dans lequel au moins l'un du premier élément de guidage (303) et du deuxième élément de guidage (303') est un anneau ou un canal partiellement ouvert dans une section transversale de celui-ci.
